(19) Europäisches Patentamt
European Patent Office
Office européen des brevets

(11) EP 4 357 340 A1

(12) **EUROPEAN PATENT APPLICATION**
published in accordance with Art. 153(4) EPC

(43) Date of publication:
**24.04.2024 Bulletin 2024/17**

(21) Application number: **22824057.8**

(22) Date of filing: **01.06.2022**

(51) International Patent Classification (IPC):
***C07D 403/12*** (2006.01) ***C07C 309/04*** (2006.01)
***C07C 303/32*** (2006.01) ***A61K 31/506*** (2006.01)
***A61P 35/00*** (2006.01)

(52) Cooperative Patent Classification (CPC):
**A61K 31/506; A61P 35/00; C07C 303/32; C07C 309/04; C07D 403/12**

(86) International application number:
**PCT/CN2022/096523**

(87) International publication number:
**WO 2022/262577 (22.12.2022 Gazette 2022/51)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.06.2021 CN 202110673090**

(71) Applicant: **Zhejiang Hisun Pharmaceutical Co., Ltd.**
**Taizhou, Zhejiang 318000 (CN)**

(72) Inventors:
- **ZHONG, Jinqing**
  **Taizhou, Zhejiang 318000 (CN)**
- **ZHANG, Xinlong**
  **Taizhou, Zhejiang 318000 (CN)**
- **GONG, Yongxiang**
  **Taizhou, Zhejiang 318000 (CN)**
- **CHEN, Lei**
  **Taizhou, Zhejiang 318000 (CN)**
- **ZHENG, Xiaohe**
  **Taizhou, Zhejiang 318000 (CN)**
- **MAO, Lifei**
  **Taizhou, Zhejiang 318000 (CN)**
- **LI, Yi**
  **Taizhou, Zhejiang 318000 (CN)**

(74) Representative: **Boult Wade Tennant LLP**
**Salisbury Square House**
**8 Salisbury Square**
**London EC4Y 8AP (GB)**

(54) **SALT OF SELECTIVE FGFR4 INHIBITOR, AND PREPARATION METHOD THEREFOR AND APPLICATION THEREOF**

(57) The present invention relates to a mesylate or ethanesulfonate salt of the compound as represented by formula (I), a preparation method therefor, a pharmaceutical composition comprising the salt, and an application of the salt.

EP 4 357 340 A1

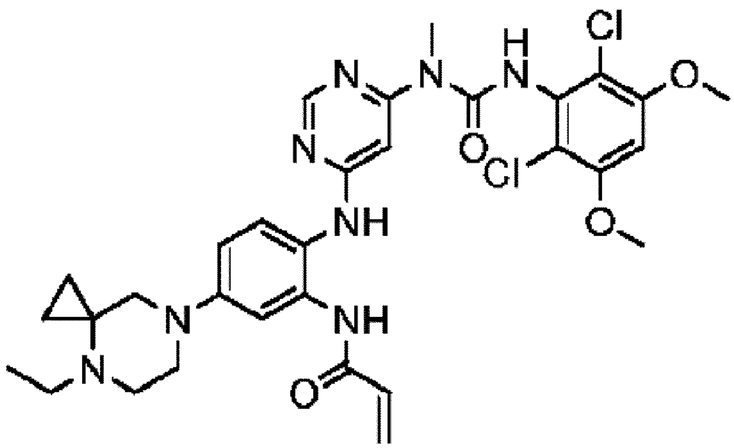

formula (I)

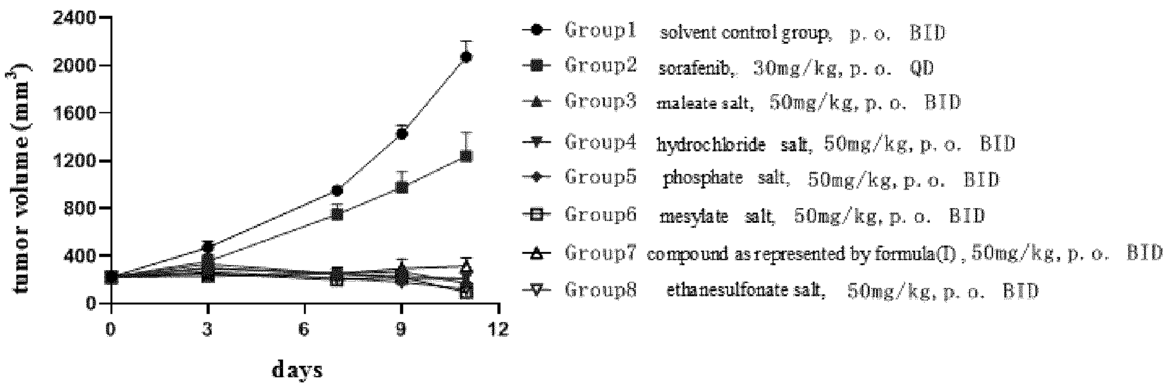


FIG 1

## Description

### CROSS REFERENCE TO RELATED APPLICATIONS

This application claims the priority of Chinese patent application No. 202110673090.0, filed with the China National Intellectual Property Administration on June 17, 2021, and titled with "SALT OF SELECTIVE FGFR4 INHIBITOR, AND PREPARATION METHOD THEREFOR AND APPLICATION THEREOF", which are hereby incorporated by reference in entirety.

### FIELD

The present invention belongs to the field of medicinal chemistry and relates to a salt of selective FGFR4 inhibitor and a preparation method thereof and applications therefor, and specifically relates to a salt of N-(2-((6-(3-(2,6-dichloro-3,5-dimethoxyphenyl)-1-methylureido)pyrimidin-4-yl)amino)-5-(4-eth yl-4,7-diazaspiro[2.5]octane-7-yl)phenyl)acrylamide and preparation method therefor, as well as a pharmaceutical composition containing the same and uses thereof.

### BACKGROUND

The patent application (PCT/CN2017/085135) reports a new class of pyrimidine derivatives, which are inhibitors against the FGFR4 pathway. A large of evidence suggests that gene amplification mutations in FGFR4 are present in lung cancer, ovarian cancer, prostate cancer, liver cancer and cholangiocarcinoma, and so on. Compared to other FGFR inhibitors, selective $FGFR_4$ inhibitors have the advantage of low toxicity (Brow, AP et al (2005), Toxcol. Pathol., 449-455). Considering the deficiency of currently available drugs in terms of safety and efficacy, and especially considering the application potential of selective $FGFR_4$ inhibitors, the current research on selective $FGFR_4$ inhibitors in anti-liver cancer and other aspects is far from enough, it is therefore desirable to investigate and develop novel $FGFR_4$ inhibitors.

PCT/CN2017/085135 reports a free-base compound as represented by formula (I) with the chemical name N-(2-((6-(3-(2,6-dichloro-3,5-dimethoxyphenyl)-1-methylureido)pyrimidin-4-yl)amino)-5-(4-eth yl-4,7-diazaspiro[2.5]octane-7-yl)phenyl) acrylamide, having the structure shown below:

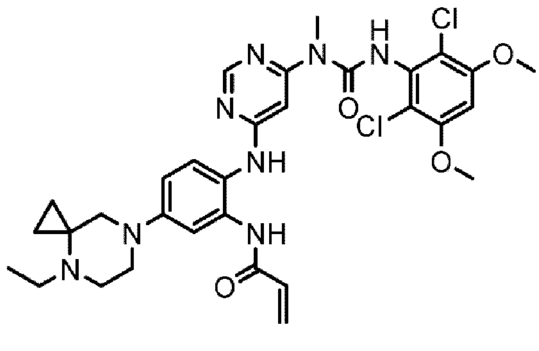

formula (I).

In vitro cell activity assay showed that a compound as represented by formula (I) has good inhibitory activity against hepatocellular carcinoma cells Hep3B with an $IC_{50}$ value of 0.019 μM, having a good prospect for development. However, in the course of investigating the druggability of the compound as represented by formula (I), the inventor of the present disclosure found that the common pharmaceutically acceptable salts of the compound as represented by formula (I) are unsatisfactory in terms of physicochemical properties and druggability, including bioavailability, water solubility, hygroscopicity, and the like. Therefore, it is necessary to conduct in-depth investigation to find new forms, being suitable for medicinal purposes, of N-(2-((6-(3-(2,6-dichloro-3,5-dimethoxyphenyl)-1-methylureido)pyrimidin-4-yl)amino)-5-(4-eth yl-4,7-diazaspiro[2.5]octane-7-yl)phenyl)acrylamide to meet the needs of drug development.

### SUMMARY

The present inventors accidentally discovered in their research that the mesylate or ethanesulfonate salts of the compound as represented by formula (I) have satisfactory druggability, including excellent water-solubility and bioavailability, and surprisingly, the mesylate and ethanesulfonate salts show significantly better effects than other salts in *in vivo* anti-tumor tests.

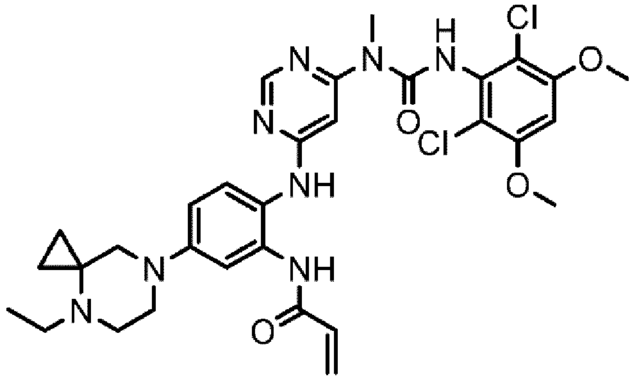

formula (I)

Based on the above findings, in a first aspect, the present disclosure provides a mesylate or ethanesulfonate salt of the compound as represented by formula (I).

Particularly preferred mesylate and ethanesulfonate salts are the mesylate salt and the ethanesulfonate salt represented by formula (I-1) and (I-2) below, respectively:

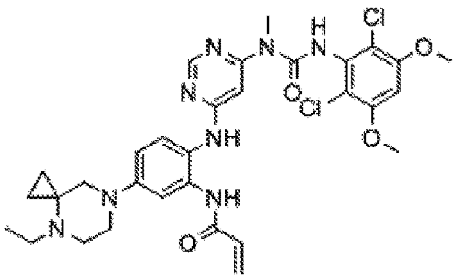

$\cdot 2CH_3SO_3H$

formula (I-1)

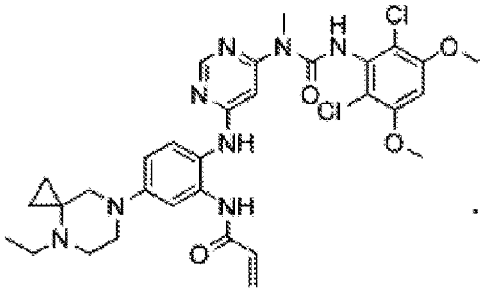

$\cdot 2C_2H_5SO_3H$

formula (I-2).

In another aspect of the present disclosure, provided is a method for preparing mesylate or ethanesulfonate salts of the compound as represented by formula (I), which comprises a step of reacting the free-base compound of formula (I) with methanesulfonic acid or ethanesulfonic acid in a solvent. In some preferred embodiments, the solvent in which the compound as represented by formula (I) is reacted with methanesulfonic acid or ethanesulfonic acid is selected from the group consisting of alcohols, ketones, methyl tert-butyl ether or a mixture thereof. In a further preferred embodiment, the solvent in which the compound as represented by formula (I) is reacted with methanesulfonic acid or

ethanesulfonic acid is selected from the group consisting of methanol, ethanol, acetone, methyl tert-butyl ether, or a mixture thereof. In a more preferred embodiment, the solvent in which the compound as represented by formula (I) is reacted with methanesulfonic acid or ethanesulfonic acid is selected from the group consisting of methanol, ethanol, acetone, methyl tert-butyl ether, or a mixture thereof.

In some specific embodiments, the compound as represented by formula (I) is reacted proportionally with methanesulfonic acid or ethanesulfonic acid in a solvent consisting of acetone and methyl tert-butyl ether at room temperature for 5 hours.

In some specific embodiments, provided is a method for preparing a mesylate or ethanesulfonate salt of the compound as represented by formula (I), comprises a step of forming a salt in a mixed solution of acetone: water = (40~10):1 containing the free base as represented by formula (I) and methanesulfonic acid or ethanesulfonic acid.

In the third aspect, the present disclosure provides a pharmaceutical composition, comprising an ethanesulfonate or mesylate salt of the compound as represented by formula (I), and a pharmaceutically acceptable carrier.

In the fourth aspect, the present disclosure provides the use of an ethylsulfonate or mesylate salt of the compound as represented by formula (I) or a pharmaceutical composition comprising the mesylate or ethylsulfonate salt in the manufacture of a medicament for use as an FGFR4 inhibitor.

The present disclosure further provides the use of an ethanesulfonate or mesylate salt of the compound as represented by formula (I) or a pharmaceutical composition comprising the mesylate or ethylsulfonate salt in the manufacture of a medicament for the treatment of a disease with $FGFR_4$ overexpression.

The present disclosure further provides the use of an ethanesulfonate or a mesylate salt of the compound as represented by formula (I) or a pharmaceutical composition comprising the mesylate or ethylsulfonate salt in the manufacture of a medicament for the treatment of a disease resulting from FGFR4 amplification.

The present disclosure further provides the use of an ethanesulfonate salt or a mesylate salt of the compound as represented by formula (I) or a pharmaceutical composition comprising the mesylate or ethylsulfonate salt in the manufacture of a medicament for the treatment of cancer, wherein the cancer is preferably selected from the group consisting of non-small cell lung cancer, gastric cancer, multiple myeloma, liver cancer, and cholangiocarcinoma, more preferably liver cancer and cholangiocarcinoma.

Accordingly, the present disclosure further provides a method, for treating a disease with FGFR4 overexpression or a disease resulting from FGFR4 amplification, which comprises administering an ethanesulfonate or a mesylate salt of the compound as represented by formula (I) of the present disclosure or a pharmaceutical composition containing the mesylate or ethanesulfonate salt to a subject in need thereof.

The present disclosure further provides a method for treating a cancer, which comprises administering an ethanesulfonate or a mesylate salt of the compound as represented by formula (I) of the present disclosure or a pharmaceutical composition containing the mesylate or ethanesulfonate salt to a subject in need thereof. Preferably, the cancer is selected from the group consisting of non-small cell lung cancer, gastric cancer, multiple myeloma, liver cancer, and cholangiocarcinoma, more preferably liver cancer and cholangiocarcinoma.

## BRIEF DESCRIPTION OF DRAWINGS

Fig. 1 shows the tumor growth curves of mice from each group in Hep3B model.

Fig. 2 is a curve graph showing changes in body weight of mice from each group in Hep3B model.

## DETAILED DESCRIPTION

The present disclosure will be explained in more detail below with reference to examples, and the examples of the present disclosure are only used to illustrate the technical embodiments of the present disclosure and do not limit the scope of the present disclosure.

For the preparation of the compound as represented by formula (I), see Example 5 of PCT/CN2017/085135, which is incorporated herein by reference in its entirety.

Preparation of the compound as represented by formula (I-1)

The free-base compound as represented by formula (I) (2000 mg, 3.0mmol) was put into a reaction bottle, and 50ml of acetone and 2.5ml of water were added, stirred well, then methanesulfonic acid (0.63g, 6.6mmol) was added, stirred until dissolution at room temperature, and the reaction solution was concentrated to remove acetone, filtered, and dried, and then 2.2 g amorphous solid was obtained with a yield of 85%.

MS m/z(ESI): 655.2321[M-$2CH_4O_3S$+H]

$^1$H NMR(400 MHz, DMSO-$d_6$)δ11.25(s, 1H), 9.73(s,1H), 9.47(s, 1H), 9.33(s, 1H), 8.45 (s, 1H), 7.39 (s, 1H), 7.34(d, *J*=8.8Hz, 1H), 6.91(s, 1H), 6.91-6.88(m,1H), 6.54-6.47(m, 2H), 6.25(d, *J*=17Hz, 1H), 5.75(d, *J*=10.4Hz, 1H), 3.94(s, 6H), 3.70-3.66(m, 1H), 3.62-3.55(m, 4H), [0026] 3.39-3.34(m, 5H), 3.12-3.09(m, 1H) , 2.41(s, 6H), 1.28-1.25(m, 5H), 1.06-0.98 (m, 2H)

Preparation of the compound as represented by formula (I-2)

The free-base compound as represented by formula (I) (1000 mg, 1.5mmol) was put into a reaction bottle, and 25ml of acetone and 1.25ml of water were added, stirred well, then ethanesulfonic acid (0.39g, 3.3mmol) was added, stirred until dissolution at room temperature, and the reaction solution was concentrated to remove acetone, filtered, and dried, and then 1.2 g amorphous solid was obtained with a yield of 90%.

MS m/z(ESI): 655.2322[M-$2C_2H_6O_3S$+H]

$^1$H NMR (400 MHz, DMSO-$d_6$) δ10.88 (brs, 1H), 9.83 (s,1H), 9.67 (s, 2H), 8.54 (s, 1H), 7.44 (s, 1H), 7.31(d, *J*=8.7Hz, 1H), 6.91-6.88(m,2H), 6.67(s, 1H), 6.51(dd, $J_1$=10.4Hz, 1H, $J_2$=16.9Hz, 1H), 6.22(d, *J*=17.1Hz, 1H), 5.73(d, *J*=10.5Hz, 1H), 3.93(s, 6H), 3.71-3.69(m, 1H), 3.61-3.54(m, 4H), 3.40 (s, 3H),3.37-3.35(m, 2H), 3.11(d, *J*=13.4Hz, 1H),2.58-2.52(m, 4H), 1.36-1.34(m, 2H), [0030] 1.28(t, *J*=6.9Hz, 3H), 1.11(t, *J*=7.4Hz, 6H) ,1.05-0.98 (m, 2H)

## Examples

### Example 1 Pharmacokinetic study on different salt forms (SD rats)

### Preparation of an administration formulation

Preparation of a formulation for gavage administration to SD rats: the free-base compound as represented by formula (I) and its mesylate salt, ethanesulfonate salt, hydrochloride salt and maleate salt were accurately weighed respectively, and an appropriate volume of 0.5% CMC-Na aqueous solution was added. After vortex oscillation, ultrasonication was carried out. If necessary, shearing and emulsification or grinding could be carried out until well mixed, to obtain an administration formulation with a concentration of 2 mg·mL$^{-1}$.

### Experimental Animals

The source and number of experimental animals are shown in Table 1. Experimental animals were kept in animal rooms, which were well ventilated and equipped with air conditioning, and the temperature was maintained at 20-25 °C, humidity at 40%-70%, and light and dark lighting for 12 hours each, and the experimental animals were allowed to eat and drink freely. After normal feeding for about 5 days, rats with good physical condition could be selected for this experiment after examination by a veterinary. Each rat was marked with a tail number.

Table 1 Source and number of experimental animals

| species | strain | source | weight (g) | number | |
|---|---|---|---|---|---|
| | | | | male | female |
| rat | Sprague Dawley (SD) | Vital River Laboratory Animal Technology Co., Ltd. | 180-300 | 30 | 0 |

### Experimental protocol

The detailed administration regimen of the animal experiment is seen in Table 2. On the day before the experiment, all SD rats were fasted overnight (equal to or more than 12 hours). On the day of the experiment, after weighing, the theoretical administration volume for each rat was calculated according to the following formula. The administration formulation should be prepared on the day of the experiment, and the interval between preparation and administration should not exceed 2 hours. The actual administration amount of each rat and the collection time of the plasma sample should be recorded in detail in the corresponding table. SD rats were allowed to restore eating 4 hours after administration, and were allowed to drink water freely during the experiment.

$$\text{Theoretical administration volume}\,(\mathrm{mL}) = \left(\frac{\text{dosage}\,(\mathrm{mg}\cdot\mathrm{kg}^{-1})}{\text{concentration of test solution}\,(\mathrm{mg}\cdot\mathrm{mL}^{-1})}\right) \times \text{weight of animals}\,(\mathrm{kg})$$

Table 2 Administration regimen of animals

| group | number | | compound | dosage ($mg \cdot kg^{-1}$) | volume ($mL \cdot kg^{-1}$) | administ ration route | course | fast or no fast | time points of blood collection |
|---|---|---|---|---|---|---|---|---|---|
| | female | male | | | | | | | |
| A | 5 | 0 | Free base | 20 | 10 | Gavage | Single | Fast | before administration and 0.25, 0.5, 1, 2, 4, 8, 12 and 24 h after administration |
| B | 5 | 0 | Ethanesulf onate salt | 20 | 10 | Gavage | Single | Fast | |
| C | 5 | 0 | Mesylate salt | 20 | 10 | Gavage | Single | Fast | |
| D | 5 | 0 | Maleate salt | 20 | 10 | Gavage | Single | Fast | |
| E | 5 | 0 | Hydrochloride salt | 20 | 10 | Gavage | Single | Fast | |

On the day of the experiment, the SD rats in group A were administered an administration formulation containing free base at a dosage of 20mg·kg$^{-1}$ by single gavage, and the SD rats in group B were administered an administration formulation containing a ethanesulfonate salt at a dosage of 20mg·kg$^{-1}$ by single gavage, and the SD rats in group C were administered an administration formulation containing a mesylate salt at a dosage of 20mg·kg$^{-1}$ by single gavage, and the SD rats in group D were administered an administration formulation containing a maleate salt at a dosage of 20mg·kg$^{-1}$ by single gavage, and the SD rats in group E were administered an administration formulation containing a hydrochloride salt at a dosage of 20mg·kg$^{-1}$ by single gavage. Before and 0.25, 0.5, 1, 2, 4, 8, 12 and 24 hours after administration, 0.15 mL blood was collected from jugular vein and placed into anticoagulant tubes containing EDTA-K2.

All whole blood samples were subjected to centrifugation for 10 minutes (5500 rpm), and then the plasma was separated and stored in a refrigerator at -30 °C to -10°C.

**Pharmacokinetic parameters**

The corresponding pharmacokinetic parameters were calculated using the non-atrioventricular model of Pharsight Phoenix 7.0, and the results are shown in Table 3 below.

Table 3 Pharmacokinetic parameters of each compound

| pharmacokinetic parameter | unit | Free base | ethanesulfonate salt | mesylate salt | maleate salt | hydrochloride salt |
|---|---|---|---|---|---|---|
| $C_{max}$ | ng·mL$^{-1}$ | 52.74 | 141.81 | 99.39 | 84.8 | 78.1 |
| $T_{max}$ | h | 4.00 | 1.50 | 0.42 | 3.00 | 3.6 |
| $T_{1/2}$ | h | 2.15 | 2.37 | 3.01 | 1.1 | NA |
| $AUC_{0-t}$ | ng·h·mL$^{-1}$ | 194.55 | 410.81 | 357.27 | 376 | 262 |

The above results show that all salt forms have significantly better pharmacokinetic properties than the free base, mainly reflected in parameters such as $AUC_{0-t}$ and $C_{max}$. Moreover, surprisingly, the pharmacokinetic properties of the mesylate and ethanesulfonate salt are significantly better than the other salt forms.

**Example 2 Study on the pharmacokinetics of different salt forms (ICR mice)**

**Preparation of an administration formulation**

Preparation of a formulation for gavage administration to ICR mice: the following tested products including mesylate salt, ethanesulfonate salt, phosphate salt, and hydrochloride salt were accurately weighed respectively, and an appropriate volume of normal saline was added. After vortex oscillation, ultrasonication was carried out. If necessary, shearing and emulsification or grinding could be carried out until well mixed, to obtain an administration formulation with a concentration of 7.5 mg·mL$^{-1}$.

**Experimental Animals**

The source and number of experimental animals are shown in Table 1. Experimental animals were kept in animal rooms, which were well ventilated and equipped with air conditioning, and the temperature was maintained at 20-25 °C, humidity at 40%-70%, and light and dark lighting for 12 hours each, and the experimental animals were allowed to eat and drink freely. After normal feeding for about 5 days, rats with good physical condition could be selected for this experiment after examination by a veterinary. Each rat was marked with a tail number.

Table 4 Source and number of experimental animals

| species | strain | source | weight (g) | number | |
|---|---|---|---|---|---|
| | | | | male | female |
| mice | ICR | Vital River Laboratory Animal Technology Co., Ltd. | 20-24 | 36 | 0 |

**Experimental protocol**

The detailed administration regimen of the ICR mice experiment is seen in Table 5. On the day before the experiment, all ICR mice were fasted overnight (equal to or more than 12 hours). On the day of the experiment, after weighing, the theoretical administration volume for each mouse was calculated according to the following formula. The administration formulation should be prepared on the day of the experiment, and the interval between preparation and administration should not exceed 2 hours. The actual administration amount of each rat and the collection time of the plasma sample should be recorded in detail in the corresponding table. ICR mice were allowed to restore eating 4 hours after administration, and were allowed to drink water freely during the experiment.

$$\text{Theoretical administration volume}\,(\mathrm{mL}) = \left(\frac{\text{dosage}\,(\mathrm{mg\cdot kg^{-1}})}{\text{concentration of test solution}\,(\mathrm{mg\cdot mL^{-1}})}\right) \times \text{weight of animals}\,(\mathrm{kg})$$

Table 5 Administration regimen of animals

| group | number | | compound | dosage ($mg \cdot kg^{-1}$) | volume ($mL \cdot kg^{-1}$) | administration route | course | fast or no fast | time points of blood collection |
|---|---|---|---|---|---|---|---|---|---|
| | female | male | | | | | | | |
| A | 9 | 0 | Phosphate salt | 75 | 10 | Gavage | Single | Fast | before administration and 0.25, 0.5, 1, 2, 4, 8, 10, 12 and 24 h after administration |
| B | 9 | 0 | Ethanesulfonate salt | 75 | 10 | Gavage | Single | Fast | |
| C | 9 | 0 | Mesylate salt | 75 | 10 | Gavage | Single | Fast | |
| D | 9 | 0 | Hydrochloride salt | 75 | 10 | Gavage | Single | Fast | |

On the day of the experiment, the ICR mice in group A were administered an administration formulation containing phosphate salt at a dose of $75mg \cdot kg^{-1}$ by single gavage, and the ICR mice in group B were administered an administration formulation containing ethanesulfonate salt at a dose of $75mg \cdot kg^{-1}$ by single gavage, and the ICR mice in group C were administered an administration formulation containing mesylate salt at a dose of $75mg \cdot kg^{-1}$ by single gavage, and the ICR mice in group D were administered an administration formulation containing hydrochloride salt at a dose of $75mg \cdot kg^{-1}$ by single gavage. Before and 0.25, 0.5, 1, 2, 4, 8, 10, 12 and 24 hours after administration, 0.15 mL blood was collected from fundus venous plexus and placed into anticoagulant tubes containing EDTA-K2.

All whole blood samples were subjected to centrifugation for 10 minutes (5500 rpm), and then the plasma was separated and stored in a refrigerator at -30 to -10 °C.

**Pharmacokinetic parameters**

The corresponding pharmacokinetic parameters were calculated using the non-atrioventricular model of Pharsight Phoenix 7.0, and the results are shown in Table 6 below.

Table 6 Pharmacokinetic parameters of each compound

| pharmacokinetic parameter | unit | phosphate salt | ethanesulfonate salt | mesylate salt | hydrochloride salt |
|---|---|---|---|---|---|
| $T_{1/2}$ | h | 2.23 | 1.37 | 1.78 | NR |
| $T_{max}$ | h | 1.00 | 0.500 | 0.250 | 2.00 |
| $C_{max}$ | $ng \cdot mL^{-1}$ | 1400 | 4110 | 3740 | 503 |
| $AUC_{0-t}$ | $ng \cdot h \cdot mL^{-1}$ | 8110 | 9660 | 8660 | 2340 |

The above results show that the mesylate and ethanesulfonate salts have significantly better pharmacokinetic properties than the other salt forms, as reflected in parameters such as $C_{max}$ and $AUC_{0-t}$.

**Example 3 Pharmacodynamic evaluation of a compound as represented formula (I) and salt-form compounds thereof in a subcutaneous transplantation model of Hep3B human liver carcinoma**

Preparation of administration formulation

Preparation of administration formulation for gavage administration: sorafenib, free-base compounds as represented by formula (I) and its maleate salts, phosphate salts, hydrochloride salts, mesylate salts, and ethanesulfonate salts were accurately weighed respectively, and an appropriate volume of normal saline was added, and then vortex oscillation was performed until mixed well, and the administration formulation was prepared just before use.

**Experimental animals**

BALB/c nude mice, female, purchased from Jiangsu Jicui Pharmacom Biotechnology Co. Licence No.: SCXK (Su) 2019-0009, Animal Qualification Certificate No.: 202102418.

After arrival, animals were required to be kept in the experimental environment for 7 days before starting the experiment. The experimental animals were kept in intelligent independent ventilation cages (IVC) with constant temperature and humidity. The temperature was maintained at 20-26 °C and the humidity was maintained at 40-70 %. Under a light-dark cycle of 10 hours light/ 14 hours dark, the experimental animals were allowed to free access to food and water. The animals were labeled using ear tag.

**Experimental regimen**

Establishment of subcutaneous tumor model of Hep3B cell: 0.2 mL of a cell suspension containing $3.5 \times 10^6$ Hep3B cells was inoculated subcutaneously on the right side of the back of female BALB/c nude mice aged over 6 weeks to establish a transplant tumor. The diameter of tumor was measured with a vernier caliper, and tumor volume was calculated using the following formula: tumor volume = $0.5 \times a \times b^2$, where a and b represented the long and short diameter of the tumor, respectively. The tumor-bearing mice were randomly divided into 9 groups and administered according to the experimental design when the average tumor volume was at a range of 200-250 $mm^3$. Mice in each treatment group and solvent control group were subjected to orally intragastric administration for 10 days. The tumor volume of mice was measured twice weekly, and the body weight was weighed daily. The curative effect was assessed

based on the relative tumor growth inhibition (TGI) rate (%), and safety was evaluated according to the change of body weight and mortality of animals.

The formulas for calculating the relative tumor volume (RTV), relative tumor proliferation rate (T/C) and relative tumor inhibition rate (TGI) are as follows:

(1) RTV (relative tumor volume) = $V_t/V_0$, where $V_0$ represents the tumor volume measured on the day of administration to groups (i.e., d0), and $V_t$ represents the tumor volume at each measurement;

(2) T/C (%) = $T_{RTV}/C_{RTV} \times 100\%$, where $T_{RTV}$ represents the RTV of a treatment group and $C_{RTV}$ indicates the RTV of the control group;

(3) TGI (%) = (1-T/C) $\times$ 100%; where T and C represents the relative tumor volume of the treatment group and control group at a specific time point, respectively.

The animal administration regimen is shown in Table 7 below.

Table 7 Table of administration regimen

| group | number of animals | group | administration dosage (mg/kg) | route of administration | frequency of administration | days after administration | administration volume (ml/kg) |
|---|---|---|---|---|---|---|---|
| 1 | 6 | Solvent control group | -- | Gavage | Twice a day | 10 | 10 |
| 2 | 6 | Sorafenib | 30 | Gavage | once a day | 10 | 10 |
| 3 | 6 | Maleate salt | 50 | Gavage | Twice a day | 10 | 10 |
| 4 | 6 | Hydrochloride salt | 50’ | Gavage | Twice a day | 10 | 10 |
| 5 | 6 | Phosphate salt | 50* | Gavage | Twice a day | 10 | 10 |
| 6 | 6 | Mesylate salt | 50* | Gavage | Twice a day | 10 | 10 |
| 7 | 6 | Free-base compound as presented by formula(I) | 50 | Gavage | Twice a day | 10 | 10 |
| 8 | 6 | Ethanesulfonate salt | 50* | Gavage | Twice a day | 10 | 10 |

Note: 1 The solvent control group was administered with saline.
2 * based on the compound as represented by formula (I).

## Experimental results

The average tumor volume of mice in the solvent control group was 2073mm$^3$ on day 11 after administration. On day 11 after administration, the average tumor volume of mice in the sorafenib treatment group was 1238mm$^3$, which was statistically significantly different than that of the control group (p=0.006), with a relative tumor growth inhibition(TGI) (%) of 42.9%. On day 11 after administration, the average tumor volume of mice in the treatment group of the compound as represented by formula (I) with maleate salt was 202mm$^3$, which was statistically significantly different from that of the control group (p<0.001), with a relative tumor inhibition(TGI) (%) of 90.7%. The average tumor volume of mice in the treatment group with hydrochloride salt of the compound as represented by formula (I) was 210mm$^3$ on day 11 after administration, which was statistically significantly different from that of the control group (p<0.001), with a relative tumor inhibition(TGI) (%) of 89.9%. The average tumor volume of mice in the treatment group with phosphate salt of the compound as represented by formula (I) was 169mm$^3$ on day 11 after administration, which was statistically significantly different from that of the control group (p<0.001), with a relative tumor inhibition(TGI) (%) of 92.1%. The average tumor volume of mice in the treatment group with mesylate salt of the compound as represented by formula (I) was 93mm$^3$ on day 11 after administration, which was statistically significantly different from that of the control group (p<0.001), with a relative tumor inhibition(TGI) (%) of 95.5%. The average tumor volume of mice in the treatment group with the compound as represented by formula (I) was 314mm$^3$ on day 11 after administration, which was statistically significantly different than that of the control group (p<0.001), with a relative tumor inhibition(TGI) (%) of 84.8%. The average tumor volume

of mice in the treatment group with ethanesulfonate salt of the compound as represented by formula (I) was $121mm^3$ on day 11 after administration, which was statistically significantly different from that of the control group ($p<0.001$), with a relative tumor inhibition(TGI) (%) of 94.0%.

As this model belongs to cachexia model, the weight of mice in the control group decreased significantly. No animals died in other treatment groups during the experiment, and the animals were well tolerated under the administered dosage.

The experimental results show that the compound as represented by formula (I) and its maleate, hydrochloride, phosphate, mesylate and ethanesulfonate salt tested in this experiment all exhibit significant anti-tumor activity and have better efficacy than sorafenib, wherein the anti-tumor activity of mesylate and ethanesulfonate salt of compound as represented by formula (I) are significantly better than that of the compound as represented by formula (I), and better than other salts. These results are particularly unexpected.

In conclusion, unexpectedly, mesylate salt and ethanesulfonate salt have significantly better anti-tumor effect compared to the other forms (free-base compound, maleate salt, hydrochloride salt, phosphate salt). As shown in the table below,, the mesylate salt group and ethanesulfonate salt group are both significantly different from the free-base compound group, and there are no significant difference in the maleate salt group, hydrochloride salt group, and phosphate salt group (Free base and other salt forms are inferior to either the mesylate salt group or the ethanesulfonate salt group in terms of tumor volume and relative tumor volume).

Table 8 Pharmacodynamic analysis of each group in Hep3B cell subcutaneous transplanted tumor model on day 11 after administration

| treatment group | tumor volume treatment ($\bar{x}\pm S$) | relative tumor volume ($\bar{x}\pm S$) | (%) | T/C (%) | P Value (compared to the control group) | P Value (compared to the group 7) |
|---|---|---|---|---|---|---|
| Group 1 (Solvent control group) | 2073±132 | 9.62±0.84 | -- | -- | -- | <0.001 |
| Group 2 (Sorafenib) 30mg/kg | 1238±199 | 5.50±0.22 | 42.90% | 57.10% | 0.006 | 0.001 |
| Group 3 (maleate salt) 50mg/kg | 202±31 | 0.90±0.12 | 90.70% | 9.30% | <0.001 | 0.184 |
| Group 4 (hydrochloride salt) 50mg/kg BID | 210±26 | 0.97±0.10 | 89.90% | 10.10% | <0.001 | 0.202 |
| Group 5 (phosphate salt) 50mg/kg BID | 169±39 | 0.79±0.17 | 92.10% | 7.90% | <0.001 | 0.105 |
| Group 6 (mesylate salt) 50mg/ kgBID | 93±32 | 0.43±0.15 | 95.50% | 4.50% | <0.001 | 0.019 |
| Group 7 (compound as represented by formula) 50mg/kg BID | 314±72 | 1.46±0.34 | 84.80% | 15.20% | <0.001 | -- |
| Group 8 (ethanesulfonate salt) 50mg/kg BID | 121±17 | 0.57±0.10 | 94.00% | 6.00% | <0.001 | 0.026 |

## Claims

1. A salt of the free base as represented by formula (I),

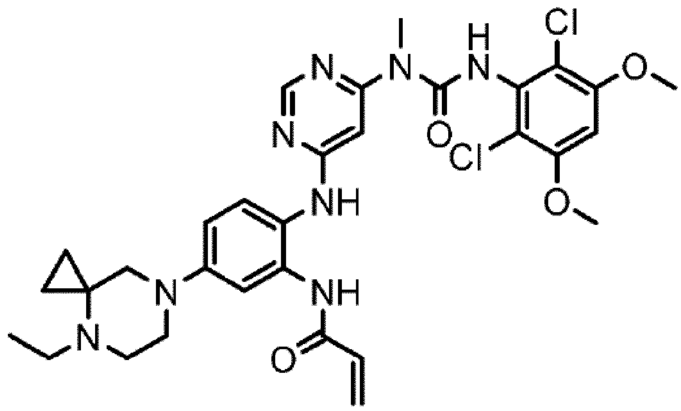

formula (I)

wherein the salt is selected from the group consisting of a mesylate salt and an ethanesulfonate salt.

2. The salt according to claim 1, which is an ethanesulfonate salt.

3. A method for preparing the salt according to claim 1 or 2, comprising a step of forming a salt in a mixed solution of acetone: water= (40-10): 1 containing the free base as represented by formula (I) and methanesulfonic acid or ethanesulfonic acid.

4. A pharmaceutical composition, comprising the salt according to any one of claims 1 to 2, and a pharmaceutically acceptable carrier, excipient or a combination thereof.

5. Use of the salt according to any one of claims 1 to 2 or of the pharmaceutical composition according to claim 4 in the preparation of a medicament for use as an FGFR4 inhibitor.

6. Use of the salt according to any one of claims 1 to 2 or of the pharmaceutical composition according to claim 4 in the preparation of a medicament for treatment of a disease with FGFR4 overexpression.

7. Use of the salt according to any one of claims 1 to 2 or of the pharmaceutical composition according to claim 4 in the preparation of a medicament for treatment of a disease resulting from FGFR4 amplification.

8. Use of the salt according to any one of claims 1 to 2 or of the pharmaceutical composition according to claim 4 in the preparation of a medicament for treatment of a cancer.

9. The use according to claim 8, wherein the cancer is selected from the group consisting of non-small cell lung cancer, gastric cancer, multiple myeloma, liver cancer and cholangiocarcinoma, preferably from liver cancer and cholangiocarcinoma.

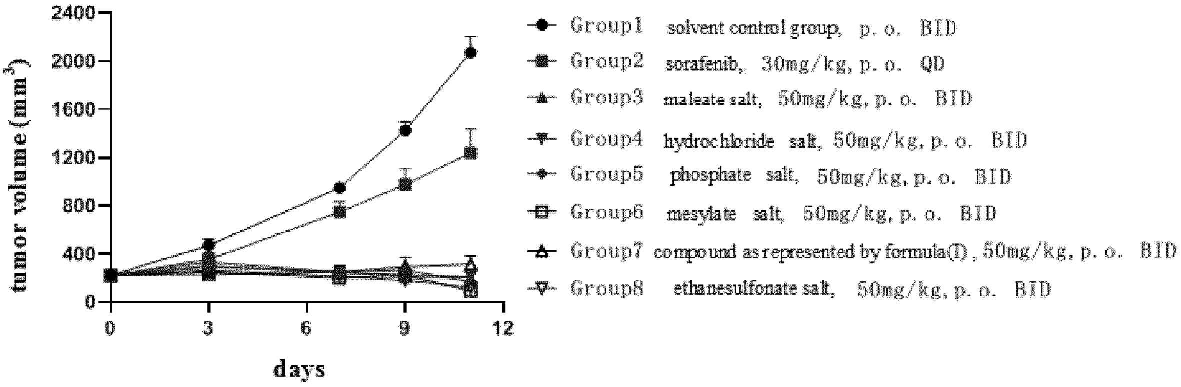
2400
2000
1600
1200
800
400
0
tumor volume (mm3)
0
3
6
9
12
days
Group1 solvent control group, p. o. BID
Group2 sorafenib, 30mg/kg, p. o. QD
Group3 maleate salt, 50mg/kg, p. o. BID
Group4 hydrochloride salt, 50mg/kg, p. o. BID
Group5 phosphate salt, 50mg/kg, p. o. BID
Group6 mesylate salt, 50mg/kg, p. o. BID
Group7 compound as represented by formula(I) , 50mg/kg, p. o. BID
Group8 ethanesulfonate salt, 50mg/kg, p. o. BID

FIG 1

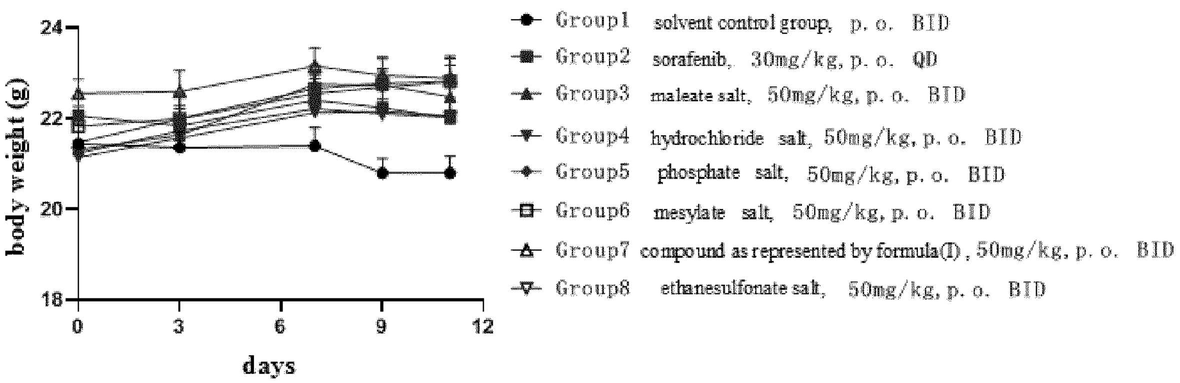
24
22
20
18
body weight (g)
0
3
6
9
12
days
Group1 solvent control group, p. o. BID
Group2 sorafenib, 30mg/kg, p. o. QD
Group3 maleate salt, 50mg/kg, p. o. BID
Group4 hydrochloride salt, 50mg/kg, p. o. BID
Group5 phosphate salt, 50mg/kg, p. o. BID
Group6 mesylate salt, 50mg/kg, p. o. BID
Group7 compound as represented by formula(I) , 50mg/kg, p. o. BID
Group8 ethanesulfonate salt, 50mg/kg, p. o. BID

FIG 2

**INTERNATIONAL SEARCH REPORT**

International application No. **PCT/CN2022/096523**

**A. CLASSIFICATION OF SUBJECT MATTER**

C07D 403/12(2006.01)i; C07C 309/04(2006.01)i; C07C 303/32(2006.01)i; A61K 31/506(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D; C07C; A61K; A61P

Documentation searched other than minimum documentation to the extent that such documents are included in the fields searched

Electronic data base consulted during the international search (name of data base and, where practicable, search terms used)

CNABS; DWPI; VEN; CNKI; CNTXT; USTXT; WOTXT; EPTXT; STN-REGISTRY; STN-CAPLUS; PATENTICS; 超星读秀, DUXIU; ISI-Web of Science: structural formula search, RN: 2155876-33-6, FGFR, 癌, 肿瘤, 肝癌, 胆管癌, 瘤, 磺酸盐, 甲磺酸盐, 乙磺酸盐, 盐

**C. DOCUMENTS CONSIDERED TO BE RELEVANT**

| Category* | Citation of document, with indication, where appropriate, of the relevant passages | Relevant to claim No. |
|---|---|---|
| A | WO 2017198221 A1 (ZHEJIANG HISUN PHARMACEUTICAL CO., LTD.) 23 November 2017 (2017-11-23)<br>description, page 5, tables, page 9, paragraph 6, page 41, table 1 | 1-9 |
| A | WO 2015057938 A1 (EISAI R&D MANAGEMENT CO., LTD. et al.) 23 April 2015 (2015-04-23)<br>entire document | 1-9 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **01 July 2022** | **15 August 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**
**Information on patent family members**

International application No.
**PCT/CN2022/096523**

| Patent document cited in search report | Publication date (day/month/year) | Patent family member(s) | Publication date (day/month/year) |
|---|---|---|---|
| WO 2017198221 A1 | 23 November 2017 | CA 3024532 A1 | 23 November 2017 |
| | | TW 201741303 A | 01 December 2017 |
| | | CN 108884097 A | 23 November 2018 |
| | | EP 3459952 A1 | 27 March 2019 |
| | | US 2019161476 A1 | 30 May 2019 |
| | | JP 2019519512 W | 11 July 2019 |
| | | TW 669300 B1 | 21 August 2019 |
| | | EP 3459952 A4 | 15 January 2020 |
| | | JP 6667014 B2 | 18 March 2020 |
| | | US 10654836 B2 | 19 May 2020 |
| | | US 2020207745 A1 | 02 July 2020 |
| | | CA 3024532 C | 09 February 2021 |
| | | US 11001572 B2 | 11 May 2021 |
| | | CN 108884097 B | 28 May 2021 |
| | | US 2021221797 A1 | 22 July 2021 |
| | | EP 3459952 B1 | 05 January 2022 |
| WO 2015057938 A1 | 23 April 2015 | CA 2924206 A1 | 23 April 2015 |
| | | TW 201605802 A | 16 February 2016 |
| | | AU 2014337291 A1 | 17 March 2016 |
| | | AR 098048 A1 | 27 April 2016 |
| | | SG 11201602069 A1 | 28 April 2016 |
| | | US 2016130237 A1 | 12 May 2016 |
| | | PH 12016500676 A1 | 30 May 2016 |
| | | KR 20160072113 A | 22 June 2016 |
| | | CN 105899490 A | 24 August 2016 |
| | | EP 3057943 A1 | 24 August 2016 |
| | | US 9434697 B2 | 06 September 2016 |
| | | VN 49093 A | 25 October 2016 |
| | | JP 2016535000 W | 10 November 2016 |
| | | MX 2016004933 A1 | 20 December 2016 |
| | | US 2017007601 A1 | 12 January 2017 |
| | | JP 6139788 B2 | 31 May 2017 |
| | | HK 1223091 A0 | 21 July 2017 |
| | | BR 112016008110 A2 | 01 August 2017 |
| | | US 9730931 B2 | 15 August 2017 |
| | | ID 201709477 A | 01 September 2017 |
| | | TW 597268 B1 | 01 September 2017 |
| | | JP 2017160233 A | 14 September 2017 |
| | | RU 2016118981 A | 23 November 2017 |
| | | US 2017360785 A1 | 21 December 2017 |
| | | KR 101826015 B1 | 06 February 2018 |
| | | ZA 201601780 A | 28 March 2018 |
| | | EP 3057943 B1 | 25 April 2018 |
| | | ES 2679521 T3 | 28 August 2018 |
| | | IL 244373 A | 31 October 2018 |
| | | HK 1223091 A1 | 09 November 2018 |
| | | AU 2014337291 B2 | 13 December 2018 |
| | | AU 2018271284 A1 | 20 December 2018 |
| | | EP 3421457 A1 | 02 January 2019 |
| | | SG 11201602069 B | 21 February 2019 |

Form PCT/ISA/210 (patent family annex) (January 2015)

**INTERNATIONAL SEARCH REPORT**
**Information on patent family members**

International application No.
**PCT/CN2022/096523**

| Patent document cited in search report | Publication date (day/month/year) | Patent family member(s) | | | Publication date (day/month/year) |
|---|---|---|---|---|---|
| | | BR | 112016008110 | B1 | 02 July 2019 |
| | | CN | 105899490 | B | 23 July 2019 |
| | | VN | 10021590 | B | 26 August 2019 |
| | | CN | 110354128 | A | 22 October 2019 |
| | | US | 10537571 | B2 | 21 January 2020 |
| | | RU | 2715708 | C2 | 03 March 2020 |
| | | AU | 2014337291 | B9 | 07 May 2020 |
| | | AU | 2018271284 | B2 | 28 May 2020 |
| | | US | 2020206222 | A1 | 02 July 2020 |
| | | IN | 202048046071 | A | 06 November 2020 |
| | | IN | 350314 | B | 06 November 2020 |
| | | MX | 378288 | B | 14 December 2020 |
| | | US | 10912774 | B2 | 09 February 2021 |
| | | NZ | 717559 | A2 | 30 July 2021 |
| | | EP | 3421457 | B1 | 06 October 2021 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202110673090
- CN 2017085135 W

### Non-patent literature cited in the description

- **BROW, AP et al.** *Toxcol. Pathol.,* 2005, 449-455